# EUROPÄISCHE PATENTSCHRIFT

(11) **EP 2 450 028 B1**
(45) Veröffentlichungstag und Bekanntmachung des Hinweises auf die Patenterteilung: **26.12.2018**
(21) Anmeldenummer: 11185194.5
(22) Anmeldetag: 14.10.2011
(51) Int. Cl.: A61Q 11/00, A61K 8/04, A61K 8/21, A61K 8/9789, A61K 8/34, A61K 8/44, A61K 8/49, A61K 8/98, A61K 36/28, A61K 36/537, A61P 29/00

(54) **Zahnpflegemittel**
Dental care agent
Produit de soin dentaire

(30) Priorität: 03.11.2010 CH 18362010
(43) Veröffentlichungstag der Anmeldung: 09.05.2012
(73) Patentinhaber: megasmile AG, 9053 Teufen (CH)
(72) Erfinder: Dr. Zettel, Roland, 9053 Teufen (CH); Kjellander, Per-Häkan, 2572 Mörigen (CH)
(74) Vertreter: Schneider Feldmann AG Patent- und Markenanwälte

(56) Entgegenhaltungen:
- EP-A2- 0 067 476
- DE-A1- 10 008 837
- JP-A- H0 225 413
- US-A1- 2005 079 140
- DATABASE GNPD [Online] MINTEL; 1. April 2007 (2007-04-01), Sodis Laboratories: "L'mastic Dent Whitening Toothpaste", XP002741761, Database accession no. 695034
- DATABASE GNPD [Online] MINTEL; 1. März 2008 (2008-03-01), GlaxoSmithKline: "Odol-Med 3 Extreme Gel Foam Tootpaste", XP002741762, Database accession no. 874480
- DATABASE WPI Week 200866 1. Juni 2008 (2008-06-01) Thomson Scientific, London, GB; AN 2008-L29123 XP002741763, & KR 2008 0006109 A (KEUM S B) 16. Januar 2008 (2008-01-16)
- SCHRADER KARLHEINZ ET AL: "Cosmetology - theory and practice : research, test methods, analysis, formulas Chapter 9.2: Toothpastes", 1. Januar 2005 (2005-01-01), COSMETOLOGY - THEORY AND PRACTICE : RESEARCH, TEST METHODS, ANALYSIS, FORMULAS, VERL. FÜR CHEMISCHE INDUSTRIE, AUGSBURG, DE, XP007906737, ISBN: 978-3-87846-245-3 * Anti-Caries Actives; Absatz [9.2.2] * * Periodontal-Prophylactic actives; Absatz [9.2.3] * * Surfactants; Absatz [9.2.8] *

## Beschreibung

Die vorliegende Erfindung betrifft ein Zahnpflegemittel zur Verwendung gegen Karies bei Patienten mit orthodentaler Behandlung in einem zur Aufschäumung geeigneten Behälter, umfassend eine wässerige Lösung mit Fluorionen freisetzendem Salz, ein zwitterionisches Schäumungsmittel und synthetischen und/oder natürlichen Aroma- und Süssstoffen.

Gegen Zahnkaries wirkende Mittel sind zwar heute durchaus gebräuchlich, trotzdem ist Karies immer noch stark verbreitet. Untersuchungen zeigen, dass überschlagsmässig immer noch rund 75% aller Kinder einen mehr oder wenige starken Kariesbefall zeigen. Daher werden insbesondere auch für Kinder speziell entwickelte Zahnpasten angeboten. Während solch übliche Zahnpasten meist durchaus für die Prophylaxe geeignet sind, trifft dies bei Personen mit orthodentaler Behandlung weniger zu. Während der orthodentalen Behandlung werden lingual oder bukkal auf den Zähnen Brackets angebracht, die über einen Arch-Wire miteinander verbunden sind. Trägt man übliche Zahnpasten auf einer Zahnbürste auf und reinigt so die Zähne, so bleiben mindestens geringe Mengen der Zahnpasta im Bereich der Brackets und unterhalb des Arch-Wire hängen und können sogar gegebenenfalls zu gewissen Ablagerungen führen, die aushärten.

Zudem treten während der orthodentalen Behandlung auch Reizungen der Schleimhäute im Mundbereich auf, und diesem Aspekt tragen herkömmliche Zahnpasten nur gering Rechnung. Der Orthodentist empfiehlt entsprechend auch meist nicht nur die Anwendung von Zahnpasten, sondern auch von Mundspülmitteln. Insbesondere die Mundspülmittel sind kaum auf Kinder ausgerichtet und haben entsprechend einer für Kindergeschmack zu intensiven Eigengeschmack.

All diese Widrigkeiten führen dazu, dass jugendliche Patienten während der Orthodentalbehandlung die Zähne nur widerwillig pflegen, obwohl während dieser Zeit eine intensivere Pflege angesagt wäre, da an den orthodentalen Vorrichtungen vermehrt Speiseresten hängen bleiben. Eine bekannte Möglichkeit Karies, insbesondere bei Kindern, zu reduzieren, besteht darin, eine geschäumte fluoride Verbindung ein- bis zweimal jährlich auf den Zähnen aufzutragen. Da dieser Schaum, der mit einem aerosolen Treibgas aufgetragen wird, soll vermieden werden, dass dieser im Mund ein Fülleeffet bewirkt, der bei diversen Patienten einen Brechreiz bewirkt.

Das Datenblatt der Zahnpaste "L'mastic Dent Whitening Toothpaste" des Herstellers "Sodis Laboratories" offenbart ein Zahnpflegemittel zur Verwendung gegen Karies umfassend eine wässrige Lösung unter anderem mit Natriumfluorid, Allantoin sowie synthetische Aroma- bzw. Süssstoffe.

Das Datenblatt der Zahnpaste "Odol-Med 3 Extreme" des Herstellers "GlaxoSmithKline" offenbart ein aufschäumbares Zahnpflegemittel zur Verwendung gegen Karies umfassend eine wässrige Lösung unter anderem mit Natriumfluorid, Cocamidopropyl Betaine als Schäumungsmittel sowie Aromen.

Das Dokument DE 100 08 337 A1 offenbart ein aufschäumbares Zahnpflegemittel zur Verwendung gegen Karies umfassend eine wässrige Lösung unter anderem mit Antikaries-Wirkstoffen auf Basis eines Fluoridsalzes sowie beispielsweise Cocamidopropylbetain als zwitterionisches Tensid.

Aus dem Dokument KR 2008/0006109 A ist ein aufschäumbares Zahnpflegemittel zur Verwendung gegen Karies bekannt umfassend eine wässrige Lösung unter anderem mit einem Fluoridsalz, Allantoin, einem Schäumungsmittel sowie Süssstoffen.

Im Weiteren ist aus Dokument US 2005/079140 A1 ein Zahnpflegemittel, insbesondere eine Zahnpasta, zur Verwendung gegen Karies bekannt, welche unter anderem Fluoridsalz und Grünteeextrakt enthält.

Aus Dokument JPH02-25413 A ist ein Zahnpflegemittel gegen Karies, welches unter anderem aus Grünteeextrakt extrahiertes Catechin enthält.

Im Weiteren ist aus der EP 0 067 476 A2 ein aufschäumbares Zahnpflegemittel umfassend unter anderem Epigallocatechin Gallate.

Im US Dokument US 2009/0087391 A wird ein aufschäumbares Zahnpflegemittel vorgeschlagen, dessen Schaum sich möglichst schnell abbaut. Aus diesem Dokument ist jedoch ein Zahnpflegemittel bekannt, mit den Merkmalen gemäss dem Oberbegriff des Patentanspruches 1.

Da diese Zusammenstellung vor allem darauf abzielt eine erhöhte Fluoreintragung im Zahnschmelz zu bewirken, kann er für die Anwendung aus Zahnpflegemittel für Patienten mit orthodentaler Behandlung nicht sinnvollerweise angewendet werden, da ein relevanter Anteil der Zahnoberfläche an den Brackets abgedeckt ist und somit hier die Fluoreintragung gar nicht erfolgen kann. Daher wird man eine Behandlung, wie sie im obgenannten Dokument angeregt ist, erst nach einer Zahnstellungskorrekturbehandlung durchführen.

Es ist daher die Aufgabe der vorliegenden Erfindung, ein Zahnpflegemittel zur Verwendung gegen Karies zu schaffen, welches besonders für Patienten mit orthodentaler Behandlung geeignet ist.

Diese Aufgabe löst ein Zahnpflegemittel zur Verwendung gegen Karies der eingangs genannten Art, welches sich dadurch auszeichnet, dass dieses zusätzlich die Gingiva pflegende und entzündungshemmende Mittel aus der Gruppe Allantoin, Propolis und Salvia Officinalis enthält sowie zusätzlich als Antioxidant und karieshemmendes Mittel Epigallocatehchin Gallate (EGCG) oder ein diesen Wirkstoff enthaltendes, natürliches Grünteeextrakt enthält, wobei ein pH-Wert von 5.5 bis 6.2 eingestellt ist und mittels eines Alkoholanteils von weniger als 3% vorhanden ist um einen stabilen feinporigen Schaum zu erzielen.

Ein solches Zahnpflegemittel ist in seiner Art eine Kombination von Zahnpasta und Mundspülemittel. Ähnlich einer herkömmlichen Zahnpasta soll hierbei während des Putzvorganges ein haltbarer Schaum im Mund verbleiben. Nur so hat der Patient das subjektive Empfinden einer korrekten Zahnpflege. Der erzeugte, stabile, feinporige Schaum vermag auch ausgezeichnet zwischen kleinsten Ritzen und Spalten einer intraoralen Dentalapplikation anzuschmiegen, ohne dass sich dabei Feststoffe daran ablagern können. Gleichzeitig mit der Reinigung findet dabei auch die entzündungshemmende Pflege der Gingiva statt.

Nachfolgend ist eine bevorzugte Zusammenstellung wiedergegeben mit deren Gewichtsprozentbereiche, und danach werden die einzelnen Komponenten der Zusammenstellung kurz bezüglich ihrer Wirkung in der Zusammenstellung beschrieben:
- A: Aqua
- D: Sorbitol
- D: Xylitol
- E: Olaflur
- E: Cocamidopropyl Betaine
- E: Alcohol
- F: Aroma
- F: Acesulfam-K
- F: Limonene
- F: Sodium Methylparaben
- F: Stevia Rebaudiana Leaf Extract (Steviol Glycoside)
- G: Allantoin
- G: Citral
- G: Propolis Extract
- G: Salvia Officinalis
- G: Epigallocatehchin Gallate (EGCG)
- G: Linalool
- G: Citric Acid

A = >50%, B = 25-50%, C = 10-25%, D = 5-10%, E = 1-5%, F = 0,1-1%, G = < 0,1%

Als Basis der Lösung dient Wasser. Hierin sind die Süssstoffe Sorbitol, Xylitol, Acesulfam-K sowie Stevia Rebaudiana Leaf Extract (Steviol Glycoside) enthalten. Zur Pflege der Mundschleimhäute und der Gingiva dienen die antiseptischen Bestandteile Alcohol, Olaflur sowie Salvia Officinalis. Cocamidopropyl Betaine wurde insbesondere gewählt als ein zwitterionisches Schäumungsmittel, das auch emulsuvierend und verdickend wirkt, und dabei einen haltbaren Schaum bewirkt.

Als antikaries wirkende Mittel wurden insbesondere Olaflur und Epigallocatehchin Gallate (EGCG) beigefügt. Epigallocatehchin Gallate ist ein Antioxidanz aus der Untergruppe der Polyphenole. Es ist in prominenter Menge im Grüntee enthalten. Neue Untersuchungen haben gezeigt, dass Epigallocatehchin Gallate auch Karies-hemmende Wirkung besitzt. Dieses Mittel ist aber bis heute in der Zahnpflege kaum berücksichtigt ganz im Gegensatz zum Olaflur das ein Dihydrofluorid eines langkettigen terziären Diamins ist und zur Stoffgruppe der Aminfluoride zählt, welches in Zahnpasten verbreitet eingesetzt wird.

Ebenfalls antiseptische und desinfizierende Wirkung, wie die eingangs erwähnten Substanzen, besitzt das Sodium-Methylparaben, welches zudem auch antibakterielle und konservierende Wirkung hat.

Allantoin wurde ebenfalls zugefügt als entzündungshemmendes und antimykotisches Mittel für die Mundschleimhäute und die Gingiva. Allantoin ist auch bekannt unter dem Namen Glyoxylsäuredioroid und hat die Summenformel C₄H₆N₄O₃.

Propolis Extract ist ein Naturgemisch vieler Inhaltsstoffe und wird aus dem sogenannten Bienenharz gewonnen. Es ist ein natürlich vorkommendes Antibiotikum, welches auch antimykotische Wirkung hat. Propolis ist insbesondere auch für die antioxidative Wirkung der antibiotischen und der cytotoxischen Eigenschaften bekannt.

Schliesslich enthält die Mischung natürliche und naturidentische Aromastoffe.

Für das hier vorliegende erfindungsgemässe Zahnpflegemittel, welches gleichzeitig die Wirkung eines Mundspülmittels besitzt, wurden verschiedene Mittel angewendet, die sowohl zahnpflegende Wirkung bzw. Antikaries-Wirkung besitzen. Diese Mittel sind insbesondere das bereits erwähnte Cocamidopropyl Betaine sowie das Epigallocatehchin Gallate. Diese kombinatorische Wirkung ist für die Verwendung von Patienten mit orthodentaler Behandlung von ausschlaggebender Bedeutung. Während die meisten Gewichtsprozent-Angaben im herkömmlichen Bereich liegen ist der geringe Alkoholanteil trotz seiner ausgezeichneten antiseptischen Wirkung ungewöhnlich. Der Alkoholanteil muss weniger als 3% betragen, da sich ansonsten die Haltbarkeit des gebildeten Schaumes drastisch reduziert.

Bevorzugterweise wird das erfindungsgemässe Gemisch in einem Behälter mit mechanischer Pumpe angeboten.

Das in der Zusammensetzung vorhandene zwitterionische Schäumungsmittel Cocamidolpropyl ist in der wässrigen Lösung emulgiert und erhöht die Oberflächenspannung um so einen feinen, haltbaren Schaum zu bilden, wenn eine Mischung von Luft und der Zusammensetzung ausgetragen wird.

Ungewöhnlich bei der vorliegenden Mischung ist auch, dass mittels der Zitronensäure der pH-Wert auf 5.6 - 5.9 eingestellt wird.

## Patentansprüche

1. Zahnpflegemittel zur Verwendung gegen Karies bei Patienten mit orthodentaler Behandlung in einem zur Aufschäumung geeigneten Behälter, umfassend eine wässerige Lösung mit Fluorionen freisetzendem Salz, ein zwitterionisches Schäumungsmittel und synthetischen und/oder natürlichen Aroma- und Süssstoffen, **dadurch gekennzeichnet, dass** dieses zusätzlich die Gingiva pflegende und entzündungshemmende Mittel aus der Gruppe Allantoin, Olaflur und Salvia Officinalis enthält sowie zusätzlich als Antioxidant und karieshemmendes Mittel Epigallocatechin Gallate (EGCG) oder ein diesen Wirkstoff enthaltender, natürlicher Grünteeextrakt enthält, wobei ein pH-Wert von 5.5 bis 6.2 eingestellt ist und mittels eines Alkoholanteils von weniger als 3% vorhanden ist um einen stabilen feinporigen Schaum zu erzielen und wobei als zwitterionisches Schäumungsmittel Cocamidopropyl Betaine gewählt wird, das emulsivierend und verdickend wirkt, und dabei einen haltbaren Schaum bewirkt.

2. Zahnpflegemittel nach Anspruch 1, **dadurch gekennzeichnet, dass** als weiteres Karies-hemmendes Mittel zusätzlich 1-5 Gew.% Olaflur zugesetzt ist.

3. Zahnpflegemittel nach Anspruch 1, **dadurch gekennzeichnet, dass** als zwitterionisches Schäumungsmittel 1-5 Gew.% Cocamidolpropyl Betaine zugesetzt ist.

4. Zahnpflegemittel nach Anspruch 1, **dadurch gekennzeichnet, dass** die Zusammensetzung natürliche und künstliche Süssstoffe aus der Gruppe: Sorbitol, Xylitol, Acesulfam-K und Stevia Rebaudiana Leaf zugefügt werden.

5. Zahnpflegemittel nach Anspruch 1, **dadurch gekennzeichnet, dass** die Zusammensetzung weniger als 0,1 Gew.% Propolis Extrakt enthält.

6. Zahnpflegemittel nach Anspruch 1, **dadurch gekennzeichnet, dass** die Zusammensetzung ein oder mehrere natürliche oder naturidentische Aromastoffe enthält.

7. Zahnpflegemittel nach Anspruch 1, **dadurch gekennzeichnet, dass** die Zusammensetzung zwischen 0,1 und 1 Gew.% Sodium Methylparaben als desinfizierendes und konservierendes Mittel zugesetzt ist.

## Claims

1. Dental care composition for use against caries by patients with orthodontic treatment in a container suitable for foaming, comprising an aqueous solution with a salt that releases fluorine ions, a zwitterionic foaming agent and synthetic and/or natural flavouring and sweetening agents, **characterized in that** it further contains the gingiva care and inflammation inhibiting substances from the group of allantoin, olaflur and salvia Officinalis, as well as the antioxidant and caries combating substance epigallocatechin gallate (EGCG) or a natural green tea extract which contains this active ingredient, wherein a pH-value from 5.5 to 6.2 is set and maintained by means of an alcohol content of less than 3% to obtain a stable, finely pored foam, and wherein cocamidopropyl betaine is selected as the zwitterionic foaming agent with emulsifying and thickening effect producing a sustained foam.

2. Dental care composition according to Claim 1, **characterized in that** 1-5 wt.% olaflur is added in addition as a further caries inhibiting substance.

3. Dental care composition according to Claim 1, **characterized in that** 1-5 wt.% cocamidopropyl betaine is added as the zwitterionic foaming agent.

4. Dental care composition according to Claim 1, **characterized in that** natural and artificial sweeteners from the group sorbitol, xylitol, acesulfame-K and stevia rebaudiana leaf are added to the composition.

5. Dental care composition according to Claim 1, **characterized in that** the composition contains less than 0.1 wt.% propolis extract.

6. Dental care composition according to Claim 1, **characterized in that** the composition contains one or more natural or nature-identical flavouring substances.

7. Dental care composition according to Claim 1, **characterized in that** between 0.1 and 1 wt.% sodium methylparaben is added to the composition as a disinfecting and conserving substance.

## Revendications

1. Produit de soin dentaire, destiné à être utilisé pour combattre les caries sur des patients en traitement orthodentaire, dans un récipient adapté au moussage, comprenant une solution aqueuse avec un sel libérant des ions de fluor, un agent moussant zwitterionique et/ou des arômes et édulcorants synthétiques et/ou naturels, **caractérisé en ce qu'**il contient en supplément des agents anti-inflammatoires et d'entretien gingival du groupe comprenant l'allantoïne, l'Olaflur et la sauge officinale, ainsi qu'additionnellement, en tant qu'antioxydant et qu'agent anticarie du gallate d'épigallocatéchine (EGCG) ou un extrait naturel de thé vert contenant ce principe actif, une valeur pH de 5.5 à 6.2 étant réglée et un pourcentage d'alcool inférieur à 3 % étant présent, pour obtenir une mousse stable fine et pour l'agent moussant zwitterionique étant choisie la bétaïne de cocamidopropyle, qui a un effet émulsifiant et épaississant et qui donne naissance à cet effet à une mousse durable.

2. Produit de soin dentaire selon la revendication 1, **caractérisé en ce qu'**en tant qu'agent anticarie supplémentaire, on ajoute par ailleurs de 1 à 5 % en poids d'Olaflur.

3. Produit de soin dentaire selon la revendication 1, **caractérisé en ce qu'**en tant qu'agent moussant zwitterionique, on ajoute de 1 à 5 % en poids de bétaïne de cocamidopropyle.

4. Produit de soin dentaire selon la revendication 1, **caractérisé en ce qu'**on ajoute à la composition des édulcorants naturels et artificiels du groupe comprenant : le sorbitol, le xylitol, l'acésulfame-K et la Stevia Rebaudiana Leaf.

5. Produit de soin dentaire selon la revendication 1, **caractérisé en ce que** la composition contient moins de 0,1 % en poids d'extrait de propolis.

6. Produit de soin dentaire selon la revendication 1, **caractérisé en ce que** la composition contient un ou plusieurs arômes naturels ou identiques au naturel.

7. Produit de soin dentaire selon la revendication 1, **caractérisé en ce qu'**on ajoute à la composition entre 0,1 et 1 % en poids de méthyl parabène sodique comme agent désinfectant et conservateur.
